Europäisches Patentamt

European Patent Office

Office européen des brevets

(19)

(11) Publication number: **0 120 619**
**B1**

# EUROPEAN PATENT SPECIFICATION

(45) Date of publication of patent specification: **30.09.87**

(21) Application number: **84301311.1**

(22) Date of filing: **29.02.84**

(51) Int. Cl.⁴: **C 07 C 1/20, B 01 J 8/24, C 10 G 3/00, C 07 C 11/02**

(54) **Process for the conversion of alcohols and oxygenates into hydrocarbons in a turbulent fluidized bed reactor.**

(30) Priority: **28.03.83 US 479521**

(43) Date of publication of application:
**03.10.84 Bulletin 84/40**

(45) Publication of the grant of the patent:
**30.09.87 Bulletin 87/40**

(84) Designated Contracting States:
**BE DE FR GB IT NL**

(56) References cited:
**EP-A-0 015 715**
**EP-A-0 099 690**
**US-A-4 229 608**
**US-A-4 238 631**
**US-A-4 328 384**
**US-A-4 338 475**

**POWDER TECHNOLOGY, vol. 24, no. 2, 1979, Lausanne; J.YERUSHALMI & N.T.CANKURT: "Further studies of the regimes of fluidization", pp. 187-205**

(73) Proprietor: **MOBIL OIL CORPORATION**
**150 East 42nd Street**
**New York New York 10017 (US)**

(72) Inventor: **Avidan, Amos Andrew**
**14 Spring Hill Road**
**Mantua New Jersey 08051 (US)**
Inventor: **Kam, Anthony Yuk-Yim**
**18 S. Birchwood Park Drive**
**Cherry Hill New Jersey 08003 (US)**

(74) Representative: **Cooper, John Anthony et al**
**Mobil Court 3 Clements Inn**
**London WC2A 2EB (GB)**

(56) References cited:
**POWDER TECHNOLOGY, vol. 7, no. 5, 1973, Lausanne; D.GELDART: "Types of gas fluidization", pp. 285-292**

**ULLMANNS "Encyklopädie der technischen Chemie", 4th edition, vol. 1, 1972, Verlag CHEMIE GmbH, Weinheim; "Allgemeine Grundlagen der Verfahrens- und Reaktionstechnik", pp. 237-239**

## Description

This invention relates to the production of light olefins, hydrocarbons boiling in the gasoline range or in the distillate range and mixtures thereof from lower aliphatic alcohols, related and other oxygenates, and mixtures thereof in a turbulent fluidized bed of ZSM-5 type zeolite catalyst.

The growing demand for fuels and light petroleum products, including light olefins and the ever decreasing reserves of crude oil, have resulted in a continuing strong interest in tapping alternative raw material sources from which to obtain the desired light hydrocarbon products.

In recent years, the patent literature has described processes in which alcohols and related oxygenates, which may be obtained from coal, natural gas or biomass, can be converted into more complex hydrocarbons, including gasoline boiling hydrocarbons, by utilizing zeolites of a group exemplified by ZSM-5 zeolite. In one commercial process natural gas is converted into gasoline; the natural gas is first steam reformed into synthesis gas which in turn is converted into methanol. The methanol is then converted into gasoline employing the known MTG (methanol to gasoline) process. The MTG process is fully described in the patent literature, for example, in U.S. Patents 3,894,103; 3,894,104; 3,894,107; 4,035,430 and 4,058,576. U.S. Patent 3,894,102 describes the conversion of synthesis gas into gasoline.

The conversion of lower monohydric alcohols into gasoline by the catalytic action of ZSM-5 type zeolites is highly exothermic. To control this exothermic reaction, there have been employed various fluidized catalyst techniques developed principally in the petroleum industry. Fluidization methods have been usefully employed in the cracking of gas oils where the powdered catalyst is used to transfer heat, developed during the regeneration of the catalyst in a fluidized bed, to another fluidized bed of catalyst to effect the endothermic cracking of the gas oil.

A number of patent publications describe the use of fluidized bed techniques not only to control the heat released in the conversion of alcohols into gasoline but also, because of the intimate contact between reactants and catalyst provided by the fluidized catalyst, to improve product selectivity and catalyst life. However, the nature of the fluidized catalyst bed employed promotes the growth of bubbles of reactant gases and the backmixing of the gaseous materials, both of which are undesirable. Different workers have addressed these problems in a variety of ways. Thus, U.S. Patent 4,071,573 describes the use of horizontally disposed grids such as wire mesh screens or heat exchangers in a dilute phase catalyst riser reactor to disperse reactant or product gas bubbles as the catalyst-reactant suspension passes upwardly through the riser; a plurality of vertical baffles or of horizontal baffles is provided in the dense phase fluid catalyst bed described in European Patents 15,716 and 15,715 which are said to restrict upflowing reactant bubble growth so as not to exceed about 15 cm and to provide substantial plug flow conditions; U.S. Patents 4,238,631 and 4,251,484 describe a dense fluidized bed reactor for methanol conversion provided with a plurality of vertical heat exchanger tubes and vertical open-ended baffle tubes which are spaced to provide a flow path having a hydraulic diameter of 10 to 20 cm when the reactants are in contact with the fluid mass of catalyst particles; and European patent application 99,690 describes the use of a dense fluidized bed reactor provided with a plurality of horizontally disposed baffles which result in an increase in the conversion efficiency as compared to an unbaffled or vertically baffled dense fluidized bed reactor.

These publications are concerned with various types of horizontal and vertical baffles for dispersing gas bubbles in the fluidized bed.

In contrast, the present invention seeks to improve the known processes by using a fluidized system which provides the gaseous reaction mixture in a series of bubbles which are small, random and short-lived, thereby providing good contact between the gaseous reactants and the catalyst particles, without the need to use baffles.

In accordance with the invention, there is provided a process for converting a $C_1$—$C_3$ aliphatic alcohol, related oxygenate and/or oxygenates produced by Fischer-Tropsch synthesis in an amount of at least 70% into a higher boiling hydrocarbon product comprising light olefins, gasoline-boiling range hydrocarbons and/or distillate boiling range hydrocarbons which comprises passing such an alcohol or oxygenate alone or in admixture with water upwardly through a fluidized bed of particles of a catalyst comprising ZSM-5 type crystalline zeolite, characterized in that the catalyst particles have a particle density of 0.6 to 2 g/cc, a particle size of up to 250 μm, an average particle size of 30 to 100 μm and a content of catalyst fines (<40 μm) of 5 to 40 weight percent and that the process is carried out at a superficial gas velocity of 0.15 to 2.15 m/s and a catalyst bed height of more than 2.4 m to maintain the catalyst bed in a state of turbulent fluidization.

The reactant of the process of the invention may be a $C_1$—$C_3$ monohydric alcohol alone or a mixture of any two or more such alcohols. The feed may also include related oxygenates and/or oxygenates produced by Fischer-Tropsch synthesis and/or water. Examples of related oxygenates are the ethers of the useful alcohols, for example, dimethyl ether (which is a related oxygenate of methanol). Water may comprise up to 20 weight percent of the feed to the process where conversions of at least 95% are desired. When partial conversions of at least 40%, preferably at least 70%, are desired, water may comprise up to about 60 weight percent of the feed.

The monohydric alcohols that may be charged to the process of the invention are methanol, ethanol, propanol and isopropanol. The feed may consist of a relatively pure single alcohol, or mixtures of these alcohols. In general, any mixture comprising methanol, ethanol, propanol, isopropanol or mixtures of any

2

two or more thereof and which is convertible with high exothermicity, is a suitable feed. Conversions which produce more than 230 KJ/kg of total hydrocarbon product, and preferably more than 460 KJ/kg of hydrocarbon product, at the conversion temperature, are considered highly exothermic for the purpose of the invention.

The preferred feed to the process of the invention is methanol optionally with dimethyl ether and up to about 20 weight percent water.

In the following description, the process of the invention is described with methanol serving as the feed; it should be understood, however, that this is done for convenience and for illustrative purposes only.

The process of the invention is carried out in the presence of a zeolite catalyst the zeolite component of which is a ZSM-5 type zeolite.

In general, the ZSM-5 type zeolites are crystalline zeolites having a silica/alumina ratio greater than 12 and a Constraint Index from 1 to 12. These zeolites and their use as conversion catalysts for lower aliphatic alcohols are described in the literature and particularly in U.S. Patents 3,894,106, 4,025,571, 4,058,576 and 4,148,835.

The preferred zeolites of the ZSM-5 type are ZSM-5, ZSM-11, ZSM-12, ZSM-23, ZSM-35, and ZSM-38, ZSM-5 being particularly preferred.

ZSM-5 is described in U.S. Patent 3,702,886; ZSM-11 is described in U.S. Patent 3,709,979; ZSM-12 is described in U.S. Patent 3,832,449; ZSM-23 is described in U.S. Patent 4,076,842; ZSM-35 is described in U.S. Patent 4,016,245; and ZSM-38 is described in U.S. Patent 4,046,859.

Especially preferred catalysts are ZSM-5 type zeolite catalysts made with large crystal ZSM-5, i.e. having a crystal size of at least 1 μm, as described in U.S. Patents 4,025,571 and 4,148,835, for example.

The feature of the invention that distinguishes the process from the known alcohol and oxygenate conversion processes discussed above is that the catalyst bed is maintained in a state of turbulent fluidization. The main advantage of this mode of operation is that the size and life span of the bubbles can be controlled, thus avoiding large scale gas by-passing in the reactor. The process of the invention does not only rely on internal baffles in the reactor for the purpose of bubble size control such as the baffles which are employed in the processes described above.

Fluidization is a phenomenon in which a bed of finely divided solid particles is lifted and agitated by a rising stream of gas. Fluidization occurs in a bed of particulate material when an upward flow of gas through the interstices of the bed of particles attains a frictional resistance equal to the weight of the bed. At this point, an incremental increase in the gas rate lifts or supports the particles. This condition is referred to as incipient bouyancy, since at this gas flow rate, the particles are still so close as to have essentially no mobility. Where it is desired to create bed homogeneity, the gas velocity can be increased to the point of blowing bubbles or voids into the bed which is then violently mixed as the bubbles rise. The increased fluid velocity at which bubbles first form is called the incipient-bubbling velocity.

Various workers have tested a wide variety of materials to study their fluidization effects. However, the conclusions drawn from data on the fluidization of one type of particulate material were found not to be applicable to other powders which had quite different particle sizes and densities. Geldart (7 *Powder Technology* 285 (1973)) has reported that the behavior of solids fluidized by gases enables those solids to be classified into four clearly recognizable groups, characterized by the difference in density between the solid particles and the fluidizing gas and the mean size of the particles. Powders in group A exhibit a dense phase expansion after minimum fluidization and prior to the commencement of bubbling, group B powders bubble at the minimum fluidization velocity; powders in group C are difficult to fluidize at all and those in group D can form stable spouted beds. The group A and B powders are those of most importance in fluidization. Fluid catalytic cracking catalyst is a typical group A powder while sand is the most typical group B powder.

The group A materials have a small mean size and/or a low particle density (usually less than about 1.4 g/cm³). Group A powders expand considerably before bubbling commences. When the gas supply is suddenly cut off the bed collapses slowly. Gross circulation of a group A powder occurs even when few bubbles are present, producing rapid mixing. Bubbles in a two-dimensional bed appeared to split and coalesce very frequently. All bubbles rise more rapidly than the interstitial gas velocity, but in freely bubbling beds the velocity of small bubbles (less than 4 cm) appears to be about 30—40 cm/s regardless of bubble size. As the superficial gas velocity is increased in a group A powder bed, slugging conditions eventually occur and with a further increase in the superficial gas velocity, slug flow breaks down into a turbulent regime. The velocity at which this turbulent regime occurs appears to decrease with particle size.

The group B powders are of less importance in fluidization. They consist of materials having a particle size of 40 to 500 μm and a density of 1.4—4 g/cm³. With group B powders bubbling starts to form at or only slightly above the minimum fluidization velocity. Bed expansion is small and the bed collapses very rapidly when the gas supply is cut off. There is little or no circulation of group B powder in the absence of bubbles. Most bubbles rise more quickly than the interstitial gas velocity. Gas velocity can be increased until slugging commences, although there is conflicting evidence regarding the breakdown of slugging into turbulent flow which takes place with group A powders.

The most easily observed difference between the group A and group B powders is whether or not the bed bubbles at or near the minimum fluidization point. If there is an appreciable bed expansion before bubbling commences, then the powder belongs to group A. Group A powders can therefore be defined as

# 0 120 619

those in which the ratio of the superficial gas velocity at minimum bubbling conditions to the superficial gas velocity at minimum fluidization is greater than one.

The turbulent regime results from increasing the gas velocity in a heterogenous, bubbling fluidized bed. The two-phase character of the bubbling bed first peaks, then gradually changes giving way to a condition of increasing uniformity culminating in the turbulent state in which large discrete bubbles or voids are on the whole absent. A bubbling fluidized bed consists of large bubbles or slugs of gas which pass upwardly through the bed of particles causing a heterogenous mixture of gas and solid particles. Like a bubbling fluidized bed, a turbulent fluidized bed has an upper bed surface although it is considerably more diffuse than in a bubbling fluidized bed because of the greater freeboard activity attending the operation at higher gas velocities. The turbulent regime extends from the transition velocity to the so-called transport velocity. (Yerushalmi et al., *Further Studies of the Regimes of Fluidization*, 24 Powder Tech. 187—205 (1979)). As the transport velocity is approached, there is a sharp increase in the rate of particle carryover, and in the absence of solid recycle, the bed would empty in a short period of time.

The transition from a bubbling fluidized bed to turbulent fluidization is gradual and spans a range of gas velocities which depends on the properties of gas and solids and on equipment scale also. The transition may be bracketed and thus characterized by two velocities: the velocity at which the pressure fluctuations peak and the velocity at which the fluctuations having decayed from their peak value and begin to level off. This velocity at the levelling off point marks the end of the transition and the onset of turbulence. Although no exact correlations exist, in general both the velocity at the peaking of pressure fluctuations and the velocity at the levelling off of the fluctuations increase with both particle size and density. (Yerushalmi et al., *supra.*).

The transition from bubbling to turbulent fluidization involves a gradual breakdown of large bubbles into small bubbles and voids. The process of bubble splitting is to some extent counterbalanced by coalescence of the resulting smaller bubbles, but the net effect is a progressive change toward a structure of greater homogeneity, culminating in the turbulent state where, on the whole, large discrete bubbles are absent. The change that takes place as the velocity of the gas spans the transition from bubbling to turbulent fluidization may be described as a process of dispersion. Large bubbles and slugs split, dispersing as smaller voids in the mass of solids. Simultaneously, the dense phase expands, but the expansion is not uniform. Instead, the solid too disperses, divided by the action of the gas phase into clusters and streamers. As a result, the structure of a turbulent fluid bed is more homogeneous consisting of two phases, of which neither can be viewed as either continuous or discontinuous.

The steady process of coalescence and splitting of the gas phase voids sets into motion a similar phenomenon in the dense phase. Together these lend the regime its "turbulent" character. Solid mixing is vigorous, the interaction of the two phases strong, and the contact between gas and solid is highly efficient. For a fluid catalytic cracking catalyst having a transfer velocity of about 1.2—1.5 m/s, the turbulent regime is from a velocity of about 0.6 m/s to a velocity of about 1.2—1.5 m/sec. Over this range, the fluidized density measured at the bottom of the bed remains rather high-ranging from about 200 to about 335 kg/m³.

Since a fluidized bed of ZSM-5 zeolite catalyst used according to the invention is operated in the turbulent regime, the gaseous reaction mixture is present as small bubbles whose existence is extremely short. The bubbles travel only a few feet as they constantly break-up and reform. Therefore when practicing the process of the invention it is unnecessary to provide baffles in the reactor vessel although such baffles are often found necessary to control the large bubbles encountered in bubbling fluidized beds, such as those employed in the prior art processes.

In order to ensure that the process of the invention occurs in the turbulent regime, several criteria concerning solid catalyst physical properties, reactor configuration and superficial fluid velocity must be satisfied simultaneously.

Table 1 below sets out essential and useful parameters required to achieve turbulent fluidization in accordance with the process of the invention. When employing a ZSM-5 type zeolite catalyst in fine powder form, such a catalyst comprises the zeolite impregnated on a suitable support with a solid density (weight of a representative individual particle divided by its apparent "outside" volume) in the range of 0.6—2 g/cc, preferably 0.9—1.6 g/cc. The catalyst particles have a wide range of particle sizes, up to 250 μm with an average particle size from 30 to 100 μm, and from 5 to 40 weight percent being constituted by fines having a particle size less than 40 μm, preferably up to 150 μm, with an average particle size from 40 to 80 μm and from 15 to 30 weight percent being constituted by fines having a particle size less than 40 μm. When these solid particles are placed in a fluidized bed where the superficial gas velocity is 0.15 to 2.15 m/s, preferably 0.24 to 0.92 m/s, operation in the turbulent regime is obtained. The velocity specified here is for an operation at a low reactor pressure of say less than 420 kPa. It will be appreciated that at higher pressures, a lower gas velocity may be employed to ensure turbulent fluidization.

The reactor can assume any commercially plausible configuration but two important criteria should be satisfied: The bed of catalyst in the reactor should be more than 2.4 m in height, preferably more than 4.5 m while the diameter of the reactor should normally be large for a commercial operation, say 2.4 to 12 m. If operation in a much smaller diameter reactor is contemplated, the superficial fluid velocity and the minimum bed height would have to be raised to ensure turbulent fluidization. These parameters will vary with the specific system in question but can be determined quite readily without undue experimentation. Removal of heat of reaction can be accomplished by the various options described in the prior art.

4

**0 120 619**

TABLE I

| Criterion | Proposed range | Preferred range |
| --- | --- | --- |
| Catalyst particle density* | 0.6—2 g/cc | 0.9—1.6 g/cc |
| Catalyst particle size range | 0—250 µm | 0—150 µm |
| Avg. catalyst particle size | 30—100 µm | 40—80 µm |
| Percent catalyst fines (<40 µm) | 5—40% | 15—30% |
| Superficial fluid velocity | 0.15—2.15 m/s | 0.24—0.92 m/s |
| Pressure | 100—700 kPa | 175—350 kPa |
| Catalyst bed height | <2.4 m | <4.5 m |
| Reactor diameter (for large scale operation) | 2.4—12 m | depends on desired throughput |

$$*\text{density} = \frac{\text{weight of one representative particle}}{\text{its apparent volume}}$$

Turbulent fluidization ensures a random, more homogenous state for the fluid bed. Bubbles are small, short-lived and in a constant state of agitation, break-up and coalescence, thereby providing good contact between the gaseous reactants and the catalyst particles. These bubbles do not travel more than a few metres in any direction in the reactor, thus minimizing gas by-passing and heterogeneity of the reactor.

Operation of the process of the invention in the turbulent fluidization regime provides a far more desirable operation than in the bubbling fluidization regime where large bubbles cause a deterioration in reactor performance.

The temperature in the fluid catalyst bed of the invention is substantially uniform. Fluid bed temperatures may range from 300 to 430°C. Usually, bed temperatures of 340 to 430°C are employed for producing gasoline while bed temperatures of 300 to 375°C are used when a highly olefinic product is desired.

In a methanol conversion process, it is preferred to achieve a methanol conversion of greater than 95% and preferably about 99.5% to minimize the loss of methanol which is difficult to recover from water formed in the process. When it is desired to change product selectivity to one of high olefin content as opposed to high aromatics' yield, the reaction time as a function of space velocity may be reduced by increasing the space velocity. On the other hand, aromatics' formation is enhanced by a reduction in reactant space velocity to increase time of contact between methanol and catalyst. A good balance in product selectivity between olefins, aromatics and paraffins may be obtained by a proper selection of reaction conditions.

**Claims**

1. A process of converting a $C_1$—$C_3$ monohydric alcohol, related oxygenate and/or oxygenates produced by Fischer-Tropsch synthesis in an amount of at least 70% into a higher boiling hydrocarbon product comprising light olefins, gasoline-boiling range hydrocarbons and/or distillate boiling range hydrocarbons which comprises passing such an alcohol or oxygenate alone or in admixture with water upwardly through a fluidized bed of particles of a catalyst comprising ZSM-5 type crystalline zeolite, characterized in that the catalyst particles have a particle density of 0.6 to 2 g/cc, a particle size of up to 250 µm, an average particle size of 30 to 100 µm and a content of catalyst fines (<40 µm) of 5 to 40 weight percent and that the process is carried out at a superficial gas velocity of 0.15 to 2.15 m/s and a catalyst bed height of more than 2.4 m to maintain the catalyst bed in a state of turbulent fluidization.

2. A process according to Claim 1, which is carried out at a pressure of 100 to 700 kPa.

3. A process according to Claims 1 and 2, wherein the catalyst particles have a particle density of 0.9 to 1.6 g/cc, a particle size of up to 150 µm, an average particle size of 30 to 100 µm and a content of catalyst fines (<40 µm) of 15 to 30 weight percent, and wherein the process is carried out at a superficial gas velocity of 0.24 to 0.92 m/s, a pressure of 175 to 350 kPa and a catalyst bed height of more than 4.5 m.

4. A process according to any one of Claims 1 to 3, wherein the alcohol is methanol.

5. A process according to any one of Claims 1 to 3, wherein the related oxygenate is dimethyl ether.

5

# 0 120 619

**Patentansprüche**

1. Verfahren zur Umwandlung eines einwertigen $C_1$—$C_3$-Alkohols, eines durch Fischer-Tropsch-Synthese hergestellten verwandten Oxidationsproduktes und/oder Oxidationsprodukte, in einer Menge von mindestens 70% in ein höher siedendes Kohlenwasserstoffprodukt, das leichte Olefine, Kohlenwasserstoffe im Benzinsiedebereich und/oder Kohlenwasserstoffe im Destillatsiedebereich umfaßt, bei dem ein solcher Alkohol oder ein solches Oxidationsprodukt allein oder in Vermischung mit Wasser nach oben durch ein Wirbelschichtbett von Partikeln eines Katalysators geführt wird, der einen kristallinen Zeolith vom Typ ZSM-5 umfaßt, dadurch gekennzeichnet, daß die Katalysatorpartikel eine Partikeldichte von 0,6 bis 2 g/cm³, eine Partikelgröße von bis zu 250 µm, eine durchschnittliche Partikelgröße von 30 bis 100 µm und einen Gehalt an Katalysatorfeinteilchen (<40 µm) von 5 bis 40 Gew.-% aufweisen und daß das Verfahren bei einer Oberflächengasgeschwindigkeit von 0,15 bis 2,15 m/s und einer Katalysatorbetthöhe von mehr als 2,4 m durchgeführt wird, um das Katalysatorbett im Zustand einer turbulenten Verwirbelung zu halten.

2. Verfahren nach Anspruch 1, daß bei einem Druck von 100 bis 700 kPa durchgeführt wird.

3. Verfahren nach Anspruch 1 oder 2, worin die Katalysatorpartikel eine Partikeldichte von 0 9 bis 1,6 g/cm³, eine Partikelgröße von bis zu 150 µm, eine durchschnittliche Partikelgröße von 30 bis 100 µm und einen Gehalt an Katalysatorfeinteilchen (<40 µm) von 15 bis 30 Gew.-% aufweisen und worin das Verfahren bei einer Oberflächengasgeschwindigkeit von 0,24 bis 0,92 m/s einen Druck von 175 bis 350 kPa und einer Katalysatorbetthöhe von mehr als 4,5 m durchgeführt wird.

4. Verfahren nach einem der Ansprüche 1 bis 3, worin der Alkohol Methanol ist.

5. Verfahren nach einem der Ansprüche 1 bis 3, worin das verwandte Oxidationsprodukt Dimethylether ist.

**Revendications**

1. Un procédé de conversion d'au moins 70% de monoalcool en $C_1$—$C_3$, un composé oxygéné apparenté et/ou de produit oxygéné formés par le synthèse de Fischer-Tropsch, en un hydrocarbure à point d'ébullition plus élevé comprenant des oléfines légères, des hydrocarbures dont les points d'ébullition sont dans la gamme de ceux l'essence et/ou des hydrocarbures dont les points d'ébullition sont dans la gamme de ceux des distillats, qui comprend le passage de bas en haut de cet alcool ou composé oxygéné, seul ou en mélange avec de l'eau, à travers un lit fluidisé de particules d'un catalyseur comportant de la zéolite cristalline du type ZSM-5, caractérisé en ce que les particules du catalysur ont une densité de particules de 0,6 à 2 g/cm³, une dimension de particules pouvant atteindre 250 µm, une dimension de particules moyenne de 30 à 100 µm et une teneur en fines de catalyseur (<40 µm) de 5 à 40% en poids, et caractérisé en ce que le procédé est mis en oeuvre à une vitesse de gaz superficielle de 0,15 à 2,15 m/s et avec une hauteur du lit de catalyseur supérieure à 2,4 m, de façon à maintenir le lit de catalyseur dans un état de fluidisation turbulente.

2. Un procédé selon la revendication 1, qui est mis en oeuvre à une pression de 100 à 700 kPa.

3. Un procédé selon les revendications 1 et 2, dans lequel les particules de catalyseur ont une densité de particules de 0,9 à 1,6 g/cm³, une dimension de particules pouvant atteindre 150 µm, une dimension de particules moyenne de 30 à 100 µm et une teneur en fines de catalyseur (<40 µm) de 15 à 30% en poids et dans lequel le procédé est mis en oeuvre à une vitesse du gaz superficielle de 0,24 à 0,92 m/s, une pression de 175 à 350 kPa et une hauteur du lit de catalyseur supérieure à 4,5 m.

4. Un procédé selon l'une quelconque des revendications 1 à 3, dans lequel l'alcool est le méthanol.

5. Un procédé selon l'une quelconque des revendications 1 à 3, dans lequel le composé oxygéne apparenté est le diméthyléther.